# EUROPEAN PATENT SPECIFICATION

(11) **EP 3 809 733 B1**
(45) Date of publication and mention of the grant of the patent: **02.12.2026**
(21) Application number: 19203764.6
(22) Date of filing: 17.10.2019
(51) Int. Cl.: H04L 9/06, H04L 9/08, H04W 12/50, H04W 12/00, H04L 9/32

(54) **SYSTEM COMPRISING A MEDICAL APPARATUS AND A REMOTE CONTROL DEVICE, METHOD FOR PAIRING THE REMOTE CONTROL DEVICE AND THE MEDICAL APPARATUS, AND METHOD FOR OPERATING THE MEDICAL APPARATUS**
SYSTEM, EINE MEDIZINISCHE VORRICHTUNG UND EINE FERNSTEUERUNGSVORRICHTUNG UMFASSEND, VERFAHREN ZUR PAARUNG DER FERNSTEUERUNGSVORRICHTUNG UND DER MEDIZINISCHEN VORRICHTUNG UND VERFAHREN ZUM BETREIBEN DER MEDIZINISCHEN VORRICHTUNG
SYSTÈME COMPRENANT UN APPAREIL MÉDICAL ET UN DISPOSITIF DE COMMANDE À DISTANCE, PROCÉDÉ D'APPARIEMENT DU DISPOSITIF DE COMMANDE À DISTANCE ET DE L'APPAREIL MÉDICAL ET PROCÉDÉ DE FONCTIONNEMENT DE L'APPAREIL MÉDICAL

(43) Date of publication of application: 21.04.2021
(73) Proprietor: Baxter Medical Systems GmbH + Co. KG, 07318 Saalfeld (DE)
(72) Inventor: LÖSER, Steffen, Batesville, IN Indiana 47006 (US); JÄGER, Matthias, Batesville, IN Indiana 47006 (US); KÖHLER, Matthias, Batesville, IN Indiana 47006 (US)
(74) Representative: Reddie & Grose LLP

(56) References cited:
- US-A1- 2014 222 574
- US-A1- 2017 093 822
- US-A1- 2017 201 380

## Description

The invention relates to a system comprising a medical apparatus and a remote control device, a method for pairing the remote control device and the medical apparatus, and a method for operating the medical apparatus, in particular for providing a distinctive wireless connection between the remote control device and the medical apparatus.

Nowadays, a plurality of medical apparatuses are operated by means of wireless remote control devices. However, in the field of the medical apparatuses, a distinctive connection between the remote control device and the medical apparatus is essential in order to prevent an unintended operation of a medical apparatus by a remote control device being not assigned to that apparatus, e.g., being located in other room, for fulfilling safety requirements. On the other hand, a hardwired configuration of a remote control device for one specific medical apparatus creates problems in operating the medical apparatus in case of replacement of the remote control device.

Therefore, a remote control device suitable for operating the medical apparatus was paired with a specific one of the medical apparatuses so that this remote control device can only operate this specific medical apparatus. However, the pairing procedure was elaborated due to the request for pressing buttons on the remote control device and on the medical apparatus.

Therefore, in order to simplify the pairing process, a pairing procedure has been developed which is initiated by merely pressing a button on the remote control device. Document EP 3 005 587 A1 discloses a pairing procedure initiated by merely pressing one button on the remote control device. Initiated by pressing the button, a pairing request is sent by the remote control device to an operating table; then, a preset device address of the operating table is then sent back from the operating table to the remote control device for establishing pairing of the remote control device and the operating table. However, due to the use of the known preset device address, the pairing between the remote control device and the operating table can easily be manipulated such that a remote control device not being intended to operate a specific operating table can be used for operating this operating table.

US 2017/093822 A1 discloses a method for enabling devices to communicate securely. A first device dynamically generates a human body nonce (HBN) and then sends that HBN to a second device via a human body communication conduit (HBCC). After sending the HBN from the first device to the second device, the first device uses the HBN to establish security for an over-the-air (OTA) communication session between the first device and the second device. For instance, the first device may derive a key, based at least in part on the HBN, and the first device may use the key to encrypt communications to be sent OTA between the first device and the second device.

Therefore, the object underlying the invention is to remedy the above-mentioned disadvantages and to provide a system of a remote control device and a medical apparatus which can be paired easily in a safe manner.

The object is achieved by a system according to claim 1, and a method for operating a medical apparatus by a remote control device according to claim 6.

Further advantageous embodiments are subject-matter of the dependent claims. According to a first aspect, the present invention provides a system according to claim 1.

By such a system, the pairing can be easily performed by sending the identifier, i.e., an identifier code, of the remote control device or of a processor of the remote control device, and a time-referenced information of the medical apparatus and by respectively creating and storing the first and the second hash key based on this identifier and this time-referenced information in the remote control device and the medical apparatus. The time-referenced information can be, e.g. a time stamp generated by the medical apparatus of a current time. The first and the second hash key can either be identical or they are not identical but have a certain relationship.

When the pairing has been successfully completed and when the instructions for controlling the medical apparatus are based on the first hash key which corresponds to the second hash key, the instructions can be transmitted in a safe manner since a distinctive connection is possible by using the unique identifier of the remote control device and the time-referenced information of the medical apparatus which can also be regarded as being unique. Moreover, since no apparatus-specific data are used for the pairing, manipulation of another remote control devices for using the established pairing is not possible since the time-referenced information is not known.

According to an advantageous implementation of the system, the remote control device comprises a switch, and the remote control device is configured to send a pairing request for initiating a pairing procedure based on the identifier of the remote control device upon an actuation of the switch.

Upon provision of such a switch, e.g., actuated by a button of the remote control device, the initiation of the pairing procedure is easily possible by pressing the button, wherein simultaneously, the identifier of the remote control device is transmitted so that no further action is necessary.

According to a further advantageous implementation of the system, the medical apparatus is configured to delete the second hash key in case of a subsequent pairing request.

By this characteristic, the use of only one remote control device for operating the medical apparatus is ensured so that no hazardous situation can occur due to an unintended operation of the medical apparatus by another remote control device.

In a further advantageous implementation of the system, the first hash key and the second hash key are identical.

When a controller of the remote control device and a controller of the medical apparatus use the same building rules for generating the hash keys, the first hash key and second hash key are identically generated in order to provide a simple procedure for generating the hash keys corresponding to each other.

By a further advantageous implementation of the system, the remote control device and the medical apparatus are configured not to send the respective hash key for itself.

When the hash keys are not sent, they cannot be detected easily and used abusively. According to a further advantageous implementation of the system, the system is configured to finish the pairing procedure when a predefined operation code is sent from the remote control device and received by the medical apparatus or when a predefined time expires.

Due to this characteristic, no further action is required for finishing the pairing procedure. For example, an easy sending of the predefined operation code from the switch or waiting until the predetermined time expires is sufficient for finishing the pairing procedure.

In a further advantageous implementation of the system, the identifier is a serial number of the remote control device.

By the use of the serial number of the remote control device, a unique identifier or identifier code can easily be determined so that a distinctive connection can easily be established.

According to a further aspect, the present invention provides a method according to claim 6.

By such a method, the pairing can be easily performed by sending the identifier of the remote control device or of the processor of the remote control device, and the time-referenced information, e.g. the time stamp of a current time of the medical apparatus, and by respectively generating the first and the second hash key based on this identifier and this time-referenced information in the remote control device and the medical apparatus.

In an advantageous implementation of the method, the method includes the step: actuating the switch of the remote control device and, thereby, sending the pairing request by the remote control device to initiate the pairing of the remote control device and the medical apparatus.

The initiation of the pairing is easily possible by actuating the switch, e.g., by pressing the button, wherein simultaneously, the identifier of the remote control device is transmitted so that no further action is necessary.

According to a further advantageous implementation of the method, the identifier of the remote control device is sent along with the pairing request for initiating the pairing.

Due to this step, the initiation of the paring is easily possible by pressing the button, wherein simultaneously, the identifier of the remote control device is transmitted so that no further action is necessary.

In a further advantageous implementation of the method, the method includes the step: finishing the pairing by sending a predefined operation code from the remote control device and receiving the predefined operation code by the medical apparatus, or by elapsing a predefined time period.

Due to this step, the pairing can be easily finished without any further action of an operator.

According to a further advantageous implementation of the method, the method includes the step: signaling the finish of the pairing by an acoustic or visual signal.

By this step, the operator is clearly informed that the pairing is finished and the remote control device can be used for operating the medical apparatus.

In another advantageous implementation of the method, the first hash key and the second hash key are identically generated.

When the identical first hash key and second hash key are generated, the controller of the remote control device and the controller of the medical apparatus can use the same building rule in order to provide a simple procedure for generating the hash keys identically.

In another advantageous implementation of the method, the second hash key is deleted by the medical apparatus in case of a subsequent pairing request.

By this step, the use of only one remote control device for operating the medical apparatus is ensured so that no hazardous situation can occur do to an unintended operation of the medical apparatus by another remote control device.

After the pairing procedure, when the instructions for controlling the medical apparatus are based on the first hash key which corresponds to the second hash key, the instructions can be transmitted in a safe manner since a distinctive connection is possible by using the unique identifier of the remote control device and the time stamp of the initiation of the pairing which is also unique. Moreover, since no apparatus-specific data are used for the pairing, manipulation of another remote control device for using the established pairing is not possible since the time stamp is not known.

Below, the invention is elucidated by means of embodiments referring to the attached drawings.

In particular,
- Fig. 1: shows a system comprising a medical apparatus and a remote control device;
- Fig. 2: shows a flow chart of method for pairing the remote control device and the medical apparatus of the system of Fig. 1; and
- Fig. 3: shows a flow chart of a method for operating the medical device by the remote control device of the system of Fig. 1.

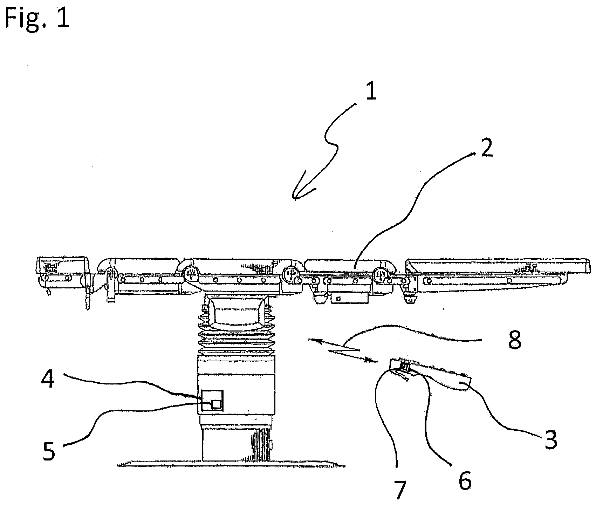

**Fig. 1** shows a system 1 comprising a medical apparatus 2 and a remote control device 3 for controlling the medical apparatus 2.

In this embodiment, the medical apparatus 2 is formed by an operating table; however, in alternative embodiments, the medical apparatus 2 can be formed by, e.g., a hospital bed or an infusion pump controlled by a respective remote control device 3.

The medical apparatus 2 is provided with a first controller 4 joined to a first transmitting-receiving device 5 and the remote control device 3 is provided with a second controller 6 joined to a second transmitting-receiving device 7. The first transmitting-receiving device 5 and the second transmitting-receiving device are configured to perform data exchange 8 with each other.

In order to be enabled to perform the data exchange 8 for operating the medical device 2 by the remote control device 3, the medical apparatus 2 and the remote control device 3 are configured to perform a pairing procedure.

The remote control device 3 comprises a switch 9 for input of an instruction for initiating the pairing procedure, i.e., for sending a paring request. In an alternative embodiment, the switch is not provided but, under specific conditions, the remote control device 3 sends a pairing request, e.g., cyclically.

The remote control device 3 is configured to send an identifier of the remote control device 3 in the pairing procedure. The identifier is a serial number of the remote control device 3; however, alternatively, a serial number of the second controller 6 or another specific identifier of the remote control device 3 can be sent.

Further, the medical device 2 is configured to send a time-referenced information. In the present embodiment, the medical device 2 sends a timestamp of a current apparatus time when receiving the pairing request. Alternatively, another time-referenced signal, e.g., a timestamp including a certain delay, can be sent. Further alternatively, the time-referenced signal is not sent when receiving the pairing request but it is sent cyclically when no pairing is established.

Furthermore, the remote control device 3 is configured to receive the time-referenced information and to generate and to store a first hash key based on the identifier of the remote control device 3 and the time-referenced information.

Moreover, the medical apparatus 2 is configured to receive the identifier of the remote control device 3 and to generate and to store a second hash key based on the identifier of the remote control device 3 and the time-referenced information.

The medical device 2 and the remote control device 3 are configured such that the second hash key generated by the medical apparatus 2 corresponds to the first hash key generated by the remote control device 3. The first hash key and the second hash key are identical. Alternatively, the first hash key and the second hash key are not identical but provided in a specific relationship.

The medical apparatus 2 and the remote control device 3 are configured not to send the respectively generated hash key as a single data set in order to avoid detection and abusively use of the hash keys. In alternative embodiment, e.g., in case of an implementation without safety-critical functions, the hash key is sent, e.g., as part of a data set of an instruction.

When the pairing procedure is successfully completed, the remote control device 3 is configured to send an instruction for controlling the medical apparatus 2 based on the first hash key and the medical apparatus 2 is configured to accept the instruction if the first hash key corresponds to the second hash key.

The remote control 3 is configured such that, based on the instruction for initiating the pairing procedure, the remote control 3 sends the pairing request for initiating the paring procedure along with the identifier of the remote control device 3 upon an actuation of the switch 9. Alternatively, the transmission of the identifier of the remote control device 3 occurs at another time.

In case that a subsequent pairing request occurs, the medical apparatus 2 is configured to delete the second hash key. This behavior enables an unambiguous correlation between the remote control device 3 and the medical apparatus 2. Alternatively, the second hash key is not deleted until a specific request for deleting the second hash key at the medical device 2 has been received.

The system is configured to finish the pairing procedure when a predefined operation code is sent from the remote control device 3 and received by the medical apparatus 2, or when a predefined time expires. Alternatively, the pairing procedure has to be finished actively by, e.g. actuating a release button.

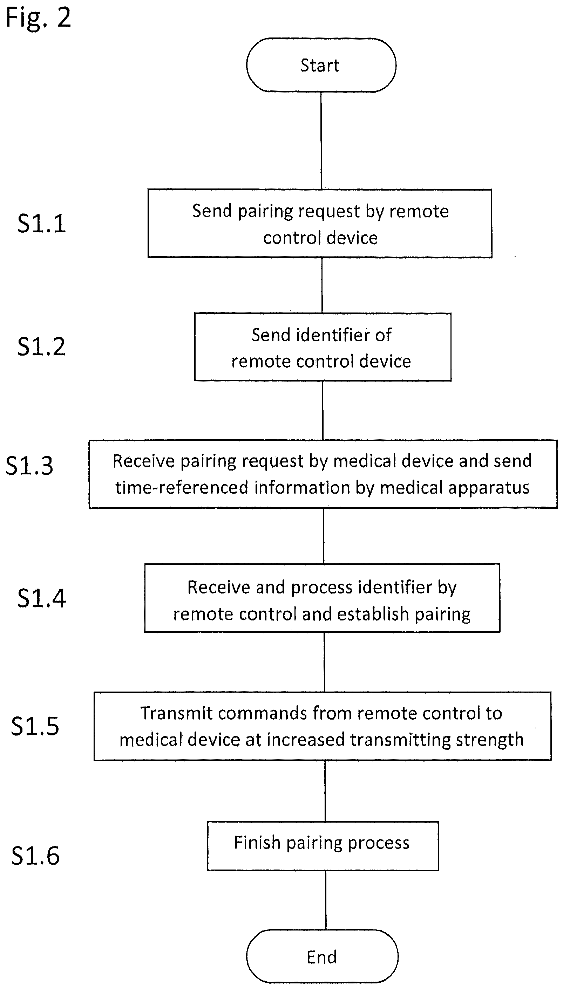

**Fig. 2** shows a flow chart of a method for pairing the remote control device 3 and the medical apparatus 2.

In use, in order to perform pairing between the remote control device 3 and the medical apparatus 2, the pairing procedure has to be initiated. This is done in step S1.1, e.g., by actuating the switch 9 of the remote control device 3 and by, thereby, sending a pairing request by the remote control device 3.

In the pairing procedure, in step S1.2, the remote control device 3 sends the identifier of the remote control device 3, as the case may be, along with the pairing request.

In Step S1.3, the medical apparatus 2 receives the pairing request and, initiated by the pairing request, sends a time-referenced information. For signaling the pairing procedure, the operating table plays a sound and all buttons of the remote control 3 are blinking. Alternatively, other signals are respectively possible.

In step S1.4, the remote control device 3 receives the time-referenced information of the medical apparatus 2 and generates a first hash key based on the identifier of the remote control device 3 and the time-referenced information.

Further, in step S1.5, the medical apparatus 2 receives the identifier of the remote control device 3 and generates a second hash key based on the identifier of the remote control device 3 and the time-referenced information. The medical apparatus 2 uses the time stamp of the current apparatus time of the medical apparatus 2 for creating the first hash key. Alternatively, a time-referenced information different from the time stamp of the current apparatus time is used.

The first hash key and the second hash key are generated by means of the same building rules and, therefore, they are identical. In another embodiment, the hash keys are not generated such that they are identical, however, they correspond to one another.

In step S1.6, the pairing procedure is finished either by sending a predefined operation code based on the first hash key from the remote control device 3 and by receiving the predefined operation code by the medical apparatus 2 or by allowing a predefined time to elapse. In this embodiment, the time is set to 30 seconds, however, alternatively another time can be set, e.g. 1 minute. In further alternative embodiments, only one of the predefined operation code and the time criteria is used. The medical apparatus 2 does not send back a confirmation signal that the pairing is completed. Alternatively, the medical apparatus 2 sends back the confirmation signal.

The finish of the pairing is signaled by an acoustic signal. In alternative embodiments, the finish of the pairing is signaled by a visual signal or by a combination of the acoustic and the visual signal.

If the pairing was successful, the buttons of the remote control device 3 stop blinking and are turned on permanently and the pairing is finished. The predefined operation code is generated by the remote control device 3 automatically as acknowledge and as indication for the medical apparatus 2 that the remote is paired. Until predefined operation code is received, the medical apparatus 2 sends the time-referenced information continuously. Alternatively, the time-referenced information is sent in another manner, e.g., cyclically.

The second hash key generated by the medical apparatus 2 is deleted by the medical apparatus 2 in case of a subsequent pairing request of anyone of a suitable remote control device 3. On the one hand, such a subsequent pairing request can be sent by the most recently paired remote control device 3, e.g. in case of a loss of the hash key by the remote control device due to pairing with another medical apparatus 2 or due to running out energy of a battery. On the other hand, the subsequent pairing request can be sent by another remote control device 3 if, e.g. the paired remote control device 3 got lost or damaged.

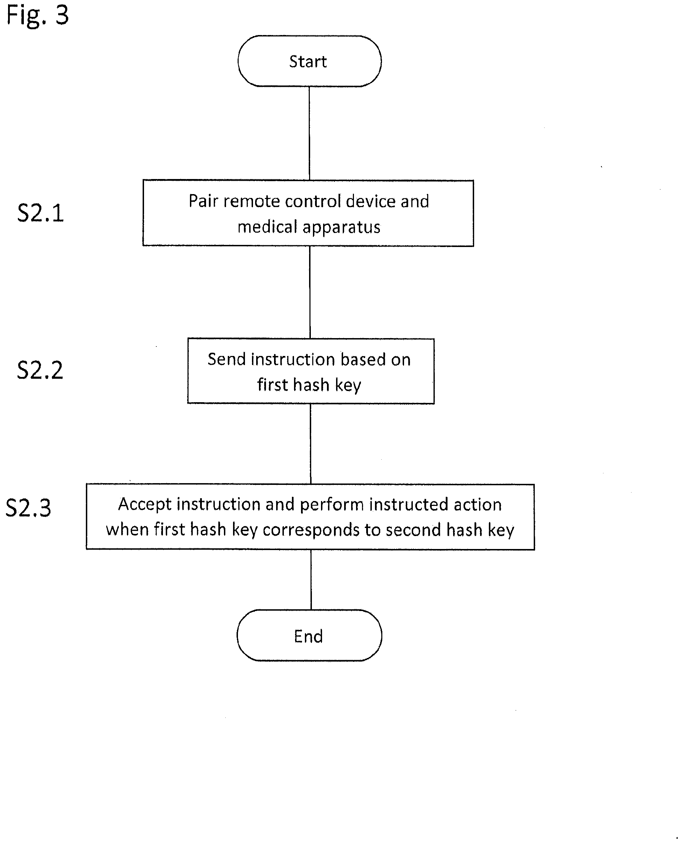

**Fig. 3** shows a flow chart of a method for operating the medical device 2 by the remote control device 3.

For operating the medical apparatus 2 by the remote control device 3, in step 2.1, the pairing of the medical apparatus 2 and the remote control device 3 is established.

After the successful pairing, when an operator instructs a certain operation of the medical apparatus 2, in step 2.2, the remote control device 3 sends an instruction based on the first hash key. The instruction is a data set including the hash key and an instruction for the certain operation of the medical apparatus 2. Alternatively, the hash key is not included but a data set according to the hash key.

In step 2.3, the medical apparatus 2 accepts the instruction when the first hash key of the remote control device 3 corresponds to the second hash key of the medical apparatus 2 and performs the instructed operation.

In case that the first hash key and the second hash key do not correspond to one another, no action of the medical apparatus 2 is performed.

While the invention has been illustrated and described in detail in the drawing and the foregoing description, such illustration and description are to be considered illustrative or exemplary and not restrictive. The invention is not limited to the disclosed embodiments. From reading the present disclosure, other modifications will be apparent to a person skilled in the art. Such modifications may involve other features, which are already known in the art and may be used instead of or in addition to features already described herein. In the claims, the word "comprising" does not exclude other elements or steps, and the indefinite article "a" or "an" does not exclude a plurality.

## Claims

1. A system (1) comprising a medical apparatus (2) and a remote control device (3) for controlling the medical apparatus (2),
the medical apparatus (2) and the remote control device (3) being configured to perform a pairing procedure, in which,
the remote control device (3) is configured to send an identifier of the remote control device (3),
the medical apparatus (2) is configured to send a time-referenced information, the remote control device (3) is configured to receive the time-referenced information and to generate and to store a first hash key based on the identifier of the remote control device (3) and the time-referenced information, and
the medical apparatus (2) is configured to receive the identifier of the remote control device (3) and to generate and to store a second hash key based on the identifier of the remote control device (3) and the time-referenced information, wherein
the medical apparatus (2) and the remote control device (3) are configured such that the second hash key corresponds to the first hash key,
wherein, when the pairing has been successfully completed, the remote control device (3) is configured to send a data set including the first hash key and an instruction for a specific operation of the medical apparatus (2) based on the first hash key, and
the medical apparatus (2) is configured to:
receive the data set including the first hash key and the instruction for the specific operation of the medical apparatus;
accept the instruction and perform the specific operation according to the instruction if the first hash key corresponds to the second hash key; and
delete the second hash key in case of a subsequent pairing request.

2. The system (1) of claim 1, wherein
the remote control device (3) comprises a switch (9), and
the remote control device (3) is configured to send a paring request for initiating a paring procedure based on the identifier of the remote control device (3) upon an actuation of the switch (9).

3. The system (1) of any preceding claim, wherein
the first hash key and the second hash key are identical.

4. The system (1) of any preceding claim, wherein
the system (1) is configured to finish the pairing procedure
when a predefined operation code is sent by the remote control device (3) and received by the medical apparatus (2), or
when a predefined time expires.

5. The system (1) of any preceding claim, wherein
the identifier is a serial number of the remote control device (3).

6. A method for operating a medical apparatus (2) by a remote control device (3), including the steps:
performing a pairing procedure between the medical apparatus and the remote control device by:
sending an identifier of the remote control device (3) by the remote control device (3);
receiving the identifier of the remote control device (3), sending a time-referenced information, and generating a second hash key based on the identifier of the remote control device (3) and the time-referenced information by the medical apparatus (2);
receiving the time-referenced information and generating a first hash key based on the identifier of the remote control device (3) and the time-referenced information by the remote control device (3), wherein the second hash key corresponds to the first hash key;
wherein when the pairing has been successfully completed:
sending a data set including the first hash key and an instruction for a specific operation of the medical apparatus (2) based on the first hash key by the remote control device (3);
receiving the data set including the first hash key and the instruction by the medical apparatus (2);
accepting the instruction by the medical apparatus (2) and performing the specific operation according to the instruction when the first hash key corresponds to the second hash key; and
deleting by the medical apparatus (2) the second hash key in case of a subsequent pairing request.

7. The method of claim 6, including the step
actuating a switch (8) of the remote control device (3) and, thereby, sending a pairing request by the remote control device (3) to initiate the pairing of the remote control device (3) and the medical apparatus (2).

8. The method of claim 7, wherein
the identifier of the remote control device (3) is sent along with the pairing request for initiating the pairing.

9. The method of anyone of claims 6 to 8, including the step
finishing the pairing procedure
by sending a predefined operation code by the remote control device (3) and receiving the predefined operation code by the medical apparatus (2), or
by elapsing a predefined time period.

10. The method of claim 9, including the step
Signaling, by the remote control device and/or the medical apparatus, the finish of the pairing by an acoustic or visual signal.

11. The method of anyone of claims 6 to 10, wherein
the first hash key and the second hash key are identically generated.

## Patentansprüche

1. System (1), umfassend eine medizinische Einrichtung (2) und eine Fernsteuerungsvorrichtung (3) zum Steuern der medizinischen Einrichtung (2),
wobei die medizinische Einrichtung (2) und die Fernsteuerungsvorrichtung (3) so konfiguriert sind, dass sie einen Paarungsvorgang durchführen, bei dem,
die Fernsteuerungsvorrichtung (3) so konfiguriert ist, dass sie eine Kennung der Fernsteuerungsvorrichtung (3) sendet,
die medizinische Einrichtung (2) so konfiguriert ist, dass sie eine zeitbezogene Information sendet,
die Fernsteuerungsvorrichtung (3) so konfiguriert ist, dass sie die zeitbezogene Information empfängt und auf der Grundlage der Kennung der Fernsteuerungsvorrichtung (3) und der zeitbezogenen Information einen ersten Hash-Schlüssel erzeugt und speichert, und
die medizinische Einrichtung (2) so konfiguriert ist, dass sie die Kennung der Fernsteuerungsvorrichtung (3) empfängt und auf der Grundlage der Kennung der Fernsteuerungsvorrichtung (3) und der zeitbezogenen Information einen zweiten Hash-Schlüssel erzeugt und speichert,
wobei
die medizinische Einrichtung (2) und die Fernsteuerungsvorrichtung (3) so konfiguriert sind, dass der zweite Hash-Schlüssel dem ersten Hash-Schlüssel entspricht,
wobei, wenn die Paarung erfolgreich abgeschlossen wurde, die Fernsteuerungsvorrichtung (3) so konfiguriert ist, dass sie einen Datensatz sendet, der den ersten Hash-Schlüssel und einen Befehl für einen spezifischen Betrieb der medizinischen Einrichtung (2) auf der Grundlage des ersten Hash-Schlüssels einschließt, und
die medizinische Einrichtung (2) so konfiguriert ist, dass sie:
den Datensatz empfängt, der den ersten Hash-Schlüssel und den Befehl für den spezifischen Betrieb der medizinischen Einrichtung einschließt;
den Befehl annimmt und den spezifischen Betrieb entsprechend des Befehls durchführt, wenn der erste Hash-Schlüssel dem zweiten Hash-Schlüssel entspricht; und
den zweiten Hash-Schlüssel im Falle einer nachfolgenden Paarungsanfrage löscht.

2. System (1) nach Anspruch 1, wobei
die Fernsteuerungsvorrichtung (3) einen Schalter (9) umfasst, und
die Fernsteuerungsvorrichtung (3) so konfiguriert ist, dass sie bei einer Betätigung des Schalters (9) eine Paarungsanfrage zum Einleiten eines Paarungsvorgangs auf der Grundlage der Kennung der Fernsteuerungsvorrichtung (3) sendet.

3. System (1) nach einem vorstehenden Anspruch, wobei
der erste Hash-Schlüssel und der zweite Hash-Schlüssel identisch sind.

4. System (1) nach einem vorstehenden Anspruch, wobei
das System (1) so konfiguriert ist, dass es den Paarungsvorgang beendet
wenn ein vordefinierter Betriebscode von der Fernsteuerungsvorrichtung (3) gesendet und von der medizinischen Einrichtung (2) empfangen wird, oder
wenn eine vordefinierte Zeit abgelaufen ist.

5. System (1) nach einem vorstehenden Anspruch, wobei
die Kennung eine Seriennummer der Fernsteuerungsvorrichtung (3) ist.

6. Verfahren zum Betreiben einer medizinischen Einrichtung (2) mittels einer Fernsteuerungsvorrichtung (3), einschließlich der Schritte:
Durchführen eines Paarungsvorgangs zwischen der medizinischen Einrichtung und der Fernsteuerungsvorrichtung durch:
Senden einer Kennung der Fernsteuerungsvorrichtung (3) durch die Fernsteuerungsvorrichtung (3);
Empfangen der Kennung der Fernsteuerungsvorrichtung (3), Senden einer zeitbezogenen Information und Erzeugen eines zweiten Hash-Schlüssels auf der Grundlage der Kennung der Fernsteuerungsvorrichtung (3) und der zeitbezogenen Information durch die medizinische Einrichtung (2);
Empfangen der zeitbezogenen Information und Erzeugen eines ersten Hash-Schlüssels auf der Grundlage der Kennung der Fernsteuerungsvorrichtung (3) und der zeitbezogenen Information durch die Fernsteuerungsvorrichtung (3), wobei der zweite Hash-Schlüssel dem ersten Hash-Schlüssel entspricht;
wobei, wenn die Paarung erfolgreich abgeschlossen wurde:
Senden eines Datensatzes einschließlich des ersten Hash-Schlüssels und eines Befehls für einen spezifischen Betrieb der medizinischen Einrichtung (2) auf der Grundlage des ersten Hash-Schlüssels durch die Fernsteuerungsvorrichtung (3);
Empfangen des Datensatzes, der den ersten Hash-Schlüssel und den Befehl einschließt, durch die medizinische Einrichtung (2);
Annehmen des Befehls durch die medizinische Einrichtung (2) und Durchführen des spezifischen Betriebs entsprechend des Befehls, wenn der erste Hash-Schlüssel dem zweiten Hash-Schlüssel entspricht; und
Löschen des zweiten Hash-Schlüssels durch die medizinische Einrichtung (2) im Falle einer nachfolgenden Paarungsanfrage.

7. Verfahren nach Anspruch 6, einschließlich des Schritts
des Betätigens eines Schalters (8) der Fernsteuerungsvorrichtung (3) und dadurch des Sendens einer Paarungsanfrage durch die Fernsteuerungsvorrichtung (3), um das Paaren der Fernsteuerungsvorrichtung (3) und der medizinischen Einrichtung (2) einzuleiten.

8. Verfahren nach Anspruch 7, wobei
die Kennung der Fernsteuerungsvorrichtung (3) zusammen mit der Paarungsanfrage zum Einleiten der Paarung gesendet wird.

9. Verfahren nach einem der Ansprüche 6 bis 8, einschließlich des Schritts
des Beendens des Paarungsvorgangs
durch das Senden eines vordefinierten Betriebscodes durch die Fernsteuerungsvorrichtung (3) und das Empfangen des vordefinierten Betriebscodes durch die medizinische Einrichtung (2) oder
durch das Verstreichen einer vordefinierten Zeitspanne.

10. Verfahren nach Anspruch 9, einschließlich des Schritts
Signalisierung des Beendens der Paarung durch die Fernsteuerungsvorrichtung und/oder die medizinische Einrichtung mittels eines akustischen oder visuellen Signals.

11. Verfahren nach einem der Ansprüche 6 bis 10, wobei
der erste Hash-Schlüssel und der zweite Hash-Schlüssel identisch erzeugt werden.

## Revendications

1. Système (1) comprenant un appareil médical (2) et un dispositif de commande à distance (3) pour commander l'appareil médical (2),
l'appareil médical (2) et le dispositif de commande à distance (3) étant configurés pour réaliser une procédure d'appariement, dans laquelle,
le dispositif de commande à distance (3) est configuré pour envoyer un identifiant du dispositif de commande à distance (3),
l'appareil médical (2) est configuré pour envoyer une information référencée temporellement,
le dispositif de commande à distance (3) est configuré pour recevoir l'information référencée temporellement et pour générer et stocker une première clé de hachage sur la base de l'identifiant du dispositif de commande à distance (3) et de l'information référencée temporellement, et
l'appareil médical (2) est configuré pour recevoir l'identifiant du dispositif de commande à distance (3) et pour générer et stocker une deuxième clé de hachage sur la base de l'identifiant du dispositif de commande à distance (3) et de l'information référencée temporellement, dans lequel
l'appareil médical (2) et le dispositif de commande à distance (3) sont configurés de sorte que la deuxième clé de hachage corresponde à la première clé de hachage,
dans lequel, lorsque l'appariement a été réalisé avec succès, le dispositif de commande à distance (3) est configuré pour envoyer un ensemble de données incluant la première clé de hachage et une instruction pour une opération spécifique de l'appareil médical (2) sur la base de la première clé de hachage, et
l'appareil médical (2) est configuré pour :
recevoir l'ensemble de données incluant la première clé de hachage et l'instruction pour l'opération spécifique de l'appareil médical ;
accepter l'instruction et réaliser l'opération spécifique selon l'instruction si la première clé de hachage correspond à la deuxième clé de hachage ; et
supprimer la deuxième clé de hachage en cas de demande d'appariement ultérieure.

2. Système (1) selon la revendication 1, dans lequel
le dispositif de commande à distance (3) comprend un commutateur (9), et
le dispositif de commande à distance (3) est configuré pour envoyer une demande d'appariement pour initier une procédure d'appariement sur la base de l'identifiant du dispositif de commande à distance (3) lors d'une activation du commutateur (9).

3. Système (1) selon une quelconque revendication précédente, dans lequel la première clé de hachage et la deuxième clé de hachage sont identiques.

4. Système (1) selon une quelconque revendication précédente, dans lequel le système (1) est configuré pour terminer la procédure d'appariement
lorsqu'un code d'opération prédéfini est envoyé par le dispositif de commande à distance (3) et reçu par l'appareil médical (2), ou
à l'expiration d'un délai prédéfini.

5. Système (1) selon une quelconque revendication précédente, dans lequel l'identifiant est un numéro de série du dispositif de commande à distance (3).

6. Procédé de commande d'un appareil médical (2) par un dispositif de commande à distance (3), incluant les étapes suivantes :
la réalisation d'une procédure d'appariement entre l'appareil médical et le dispositif de commande à distance par :
l'envoi d'un identifiant du dispositif de commande à distance (3) par le dispositif de commande à distance (3) ;
la réception de l'identifiant du dispositif de commande à distance (3), l'envoi d'une information référencée temporellement, et la génération d'une deuxième clé de hachage sur la base de l'identifiant du dispositif de commande à distance (3) et de l'information référencée temporellement par l'appareil médical (2) ;
la réception de l'information référencée temporellement et la génération d'une première clé de hachage sur la base de l'identifiant du dispositif de commande à distance (3) et de l'information référencée temporellement par le dispositif de commande à distance (3), dans lequel la deuxième clé de hachage correspond à la première clé de hachage ;
dans lequel lorsque l'appariement a été terminé avec succès :
l'envoi d'un ensemble de données incluant la première clé de hachage et une instruction pour une opération spécifique de l'appareil médical (2) sur la base de la première clé de hachage par le dispositif de commande à distance (3) ;
la réception de l'ensemble de données incluant la première clé de hachage et l'instruction par l'appareil médical (2) ;
l'acceptation de l'instruction par l'appareil médical (2) et la réalisation de l'opération spécifique selon l'instruction lorsque la première clé de hachage correspond à la deuxième clé de hachage ; et
la suppression par l'appareil médical (2) de la deuxième clé de hachage en cas de demande d'appariement ultérieure.

7. Procédé selon la revendication 6, incluant l'étape d'actionnement d'un commutateur (8) du dispositif de commande à distance (3) et, ainsi, d'envoi d'une demande d'appariement par le dispositif de commande à distance (3) pour initier l'appariement du dispositif de commande à distance (3) et de l'appareil médical (2).

8. Procédé selon la revendication 7, dans lequel
l'identifiant du dispositif de commande à distance (3) est envoyé conjointement avec la demande d'appariement pour initier l'appariement.

9. Procédé selon l'une quelconque des revendications 6 à 8, incluant l'étape de terminaison de la procédure d'appariement
par l'envoi, par le dispositif de commande à distance (3), d'un code d'opération prédéfini et la réception, par l'appareil médical (2), du code d'opération prédéfini, ou
par l'écoulement d'une période de temps prédéfinie.

10. Procédé selon la revendication 9, incluant l'étape de signalisation, par le dispositif de commande à distance et/ou l'appareil médical, de la terminaison de l'appariement par un signal acoustique ou visuel.

11. Procédé selon l'une quelconque des revendications 6 à 10, dans lequel
la première clé de hachage et la deuxième clé de hachage sont générées de manière identique.
